# EUROPEAN PATENT SPECIFICATION

(11) **EP 2 921 166 B1**
(45) Date of publication and mention of the grant of the patent: **14.06.2017**
(21) Application number: 13854991.0
(22) Date of filing: 15.11.2013
(51) Int. Cl.: A61K 31/136, A61K 31/7068, A61K 9/16, A61K 9/14, A61K 47/40, A61K 38/38, A61K 31/704, A61K 31/4745

(54) **BIODEGRADABLE MICROBEADS WITH IMPROVED ANTICANCER DRUG ADSORPTIVITY, CONTAINING ALBUMIN AND DEXTRAN SULFATE, AND PREPARATION METHOD THEREFOR**
BIOLOGISCH ABBAUBARE MIKROKÜGELCHEN MIT VERBESSERTER ADSORBIERBARKEIT VON KREBSMEDIKAMENTEN MIT ALBUMIN UND DEXTRANSULFAT SOWIE HERSTELLUNGSVERFAHREN DAFÜR
MICROBILLES BIODÉGRADABLES AYANT UNE CAPACITÉ D'ADSORPTION DE MÉDICAMENT ANTICANCÉREUX AMÉLIORÉE CONTENANT DE L'ALBUMINE ET DU SULFATE DE DEXTRAN, ET LEUR PROCÉDÉ DE PRÉPARATION

(30) Priority: 15.11.2012 KR 20120129722
(43) Date of publication of application: 23.09.2015
(73) Proprietor: Utah-Inha DDS & Advanced Therapeutics Research Center, Yeonsu-gu, Incheon 406-840 (KR)
(72) Inventor: KIM, Se Yoon, Siheung-si Gyeonggi-do 429-440 (KR); LEE, Don Haeng, Seoul 140-011 (KR); LI, Yixian, Yeonsu-gu Incheon 406-735 (KR)
(74) Representative: Brevalex
(86) International application number: PCT/KR2013/010419
(87) International publication number: WO 2014/077629

(56) References cited:
- EP-A1- 2 926 806
- WO-A1-95/13798
- KR-A- 20050 042 507
- CHEN, Y. ET AL: 'SYNTHESIS OF ALBUMIN-DEXTRAN SULFATE MICROSPHERES POSSESSING FAVOURABLE LOADING AND RELEASE CHARACTERISTICS FOR THE ANTICANCER DRUG DOXORUBICIN?' JOURNAL OF CONTROLLED RELEASE vol. 31, no. 1, August 1994, pages 49 - 54, XP025526742 DOI: 10.1016/0168-3659(94)90250-X
- CHANGGUANG WANG ET AL: 'PINGYANGMYCIN LOADED BOVINE SERUM ALBUMIN MICROSPHERES FOR CHEMOEMBOLIZATION THERAPY IN VITRO AND IN VIVO STUDIES' INTERNATIONAL JOURNAL OF PHARMACEUTICS vol. 351, no. 1-2, 03 March 2008, pages 219 - 226, XP022455536 DOI: 10.1016/J.IJPHARM.2007.09.045
- H.F.M. CREMERS ET AL: 'ALBUMIN-HEPARIN MICROSPHERES AS CARRIERS FOR CYTOSTATIC AGENTS' JOURNAL OF CONTROLLED RELEASE vol. 11, no. 1-3, 1990, pages 167 - 179, XP023744017 DOI: 10.1016/0168-3659(90)90130-L

## Description

### Technical Field

The present invention relates to biodegradable microbeads with improved anticancer drug adsorptivity, and a method for preparing the same, and a method for treating cancer using the same.

### Background Art

Recent development of imaging technologies can locate cancer that is hiding in the body, and thus the cancer can be removed by several methods such as radiation irritation and endoscopy operation. However, even though the exact location of the cancers is founded, the surgical exclusion of the cancers is impossible due to several reasons, such as the cancer spreading out all over the whole organs or adjoining to another organ. Liver cancer, pancreatic cancer, or the like, even though detected, cannot be radically cured through surgical operation.

Currently, transarterial chemoembolization (TACE), which is most commonly done in the treatment of a liver tumor, is a treatment wherein an anticancer drug is administered to the artery which supplies nutrition to the liver tumor, and then the blood vessel is blocked. Liver tissues receive oxygen and nutrients through the portal vein which turns around the small intestine and large intestine, and the hepatic artery, which comes out directly from the main artery. Normal live tissues receive blood from mainly the portal vein, and the tumor tissues receive blood from mainly the hepatic artery. Therefore, in cases where an anticancer drug is administered to the hepatic artery, which supplies nutrition to the tumor, and then the blood vein is blocked, only the tumor can be selectively necrotized without harming normal liver tissues. Such a treatment has many advantages, such as having no restrictions according to the progression of cancer and thus having a wide range of applications, and having a few limitations in the objects of the treatment, and thus currently makes a large contribution on the improvement in the cure rate of the liver cancer. As for chemoembolization, a catheter is first inserted into the femoral artery in the groin and approaches the hepatic artery, and then a vascular contrast medium is injected to obtain information necessary for the treatment, such as positions, sizes, and blood supply aspects of tumors. When the treatment protocol is decided, a thin tube with a thickness of about 1 mm is inserted into the catheter, and then the artery to be targeted is found, followed by surgical operation.

Currently, representatively, hepatic embolization using lipiodol has been clinically applied most frequently, and a significant number of patent technologies using the hepatic embolization have also been reported. Lipiodol contains a lot of iodine as a constituent element, and thus allows CT imaging, thereby providing a convenient surgical procedure. However, in order to load doxorubicin, an injection in which a drug is dissolved needs to be shaken and mixed with oily lipiodol immediately before the surgical operation. In addition, it has been clinically reported that after the surgical operation, the doxorubicin dissolved in an aqueous phase does not accumulate in the liver cancer site, but promptly leaks into the body blood, thereby failing to obtain a sufficient anticancer effect and causing a considerable side effect to patients.

U.S. Patent No. 7,442,385 discloses a method wherein, after polyvinylalcohol (PVA) is cross-linked to prepare micro-sized particles, doxorubicin as a cancer drug is adsorbed on surfaces of beads via an electric attraction and then transferred to the liver cancer site, thereby attaining both a sustained release of anticancer drug and an embolization effect. For achieving this, during a cross-linkage procedure of polyvinylalcohol, 2-acrylamido-2-methylpropane sulfonic acid (AMPS), which is an anionic monomer, is covalently linked to the end of the cross-linkage to modify the polymer, thereby allowing the polymer to adsorb an anionic drug, such as doxorubicin. However, according to the hepatic embolization using polyvinylalcohol, cross-liked PVA does not degrade in the body, and thus, after the necrotization of the liver tumor, PVA beads were irregularly diffused in the body, causing an inflammation, or more unfortunately, the PVA beads go down the blood vessel and spreads into another organ, causing cerebrovascular disease. Therefore, a drug delivery system capable of achieving both a function as an anticancer drug carrier and a vascular embolization function to solve the foregoing problems is required. WO 95/13798 A1 and CHEN, Y. ET AL ("SYNTHESIS OF ALBUMIN-DEXTRAN SULFATE MICROSPHERES POSSESSING FAVOURABLE LOADING AND RELEASE CHARACTERISTICS FOR THE ANTICANCER DRUG DOXORUBICIN?", JOURNAL OF CONTROLLED RELEASE, vol. 31, no. 1, August 1994 (1994-08), pages 49-54, DOI: 10.1016/0168-3659(94)90250-X) describe describe microbeads comprising dextran and chemically crosslinked albumin. In addition, a chemical cross-linking agent, such as glutaraldehyde, is normally used when the microbeads are prepared. In cases where the cross-linking is conducted by using glutaraldehyde, the inflow of glutaraldehyde into the body causes a risk of tissue fibrosis, and thus the residual glutaraldehyde needs to be completely neutralized using 5% sodium hydrogen sulfite, and then be washed with distilled water several times. Therefore, due to the glutaraldehyde removing process, the preparation process of microbeads using glutaraldehyde is complex and non-economical, and the anticancer drug adsorbed on the beads as well as glutaraldehyde is also removed, thereby ultimately lowering the adsorption rate of the anticancer drug.

Throughout this application, various patents and publications are referenced and citations are provided in parentheses. The disclosure of these patents and publications in their entities are hereby incorporated by references into this application in order to more fully describe this invention and the state of the art to which this invention pertains.

### Detailed Description of the Invention

### Technical Problem

The present inventors have endeavored to develop a method for preparing microbeads, capable of solving the problem in that existing microbeads for the local treatment of cancer do not degrade in the body, allowing large amounts of an anticancer drug to be adsorbed onto microbeads, and simplifying the preparing process to improve economic efficiency. As a result, albumin was used as a polymer material for forming a shape of a bead, and microbeads were prepared such that dextran sulfate, which is an anionic polymer, was included in an albumin cross-linked product so as to allow an anticancer drug to be adsorbed onto surfaces of the beads, and thus the present invention was completed. Further, as a result of preparing microbeads in which albumin was thermally cross-linked and then checking the anticancer drug adsorptivity of the microbeads, it was confirmed that the anticancer drug adsorptivity of the present microbeads were remarkably superior to that of beads prepared by using glutaraldehyde as a cross-linking agent, and thus the present invention has been completed.

Therefore, an aspect of the present invention is to provide biodegradable microbeads with improved anticancer drug adsorptivity.

Another aspect of the present inventio is to provide a method for preparing biodegradable microbeads with improved anticancer drug adsorptivity.

Still another aspect of the present invention is to provide microbeads for treating cancer.

Other objects and advantages of the present invention will become apparent from the detailed description to follow taken in conjunction with the appended claims and drawings.

### Technical Solution

In accordance with an aspect of the present invention, there is provided biodegradable microbeads with improved anticancer drug adsorptivity, the microbead including:
(i) albumin which is cross-linked by thermal cross-linkage to form a shape of a bead; and
(ii) dextran sulfate, as an anionic polymer, included in the albumin cross-linked product.

The present inventors have endeavored to develop a method for preparing microbeads, capable of solving the problem in that existing microbeads for the local treatment of cancer do not degrade in the body, allowing large amounts of an anticancer drug to be adsorbed onto microbeads, and simplifying the preparing process to improve economic efficiency. As a result, albumin was used as a polymer material for forming a shape of a bead, and microbeads were prepared such that dextran sulfate, which is an anionic polymer, was included in an albumin cross-linked product so as to allow an anticancer drug to be adsorbed onto surfaces of the beads. Further, as a result of preparing microbeads in which albumin was thermally cross-linked and then checking the anticancer drug adsorptivity of the microbeads, it was confirmed that the anticancer drug adsorptivity of the present microbeads were remarkably superior to that of beads prepared by using glutaraldehyde as a cross-linking agent.

According to one embodiment of the present invention, the microbeads further comprise an anticancer drug adsorbed onto a bead surface by an electrostatic attraction with the anionic polymer.

In a specific embodiment, the anticancer drug is an anthracycline based anticancer drug. Examples of the anthracycline based anticancer drug are doxorubicin, daunorubicin, epirubicin, idarubicin, gemcitabine, mitoxantrone, pararubicin, and valrubicin

In another specific embodiment, the anticancer drug is irinotecan.

According to one embodiment of the present invention, the microbeads of the present invention are microbeads for use in chemoembolization for the treatment of solid cancer.

In one specific embodiment, the microbeads of the present invention are beads for chemoembolization for liver cancer (hepatic artery embolization). As for the solid cancer to which embolization is applicable besides the treatment of liver cancer, rectal cacinom may be treated through rectal artery (K. Tsuchiya, Urology, 55(4):495-500 (2000)).

The microbeads of the present invention include, as constituent elements thereof, albumin and dextran sulfate. The albumin is thermally cross-linked to function as a support for forming and maintaining the shape of a microbead. The dextran sulfate, which is an anionic polymer, is included in the cross-linked albumin to allow an anticancer drug to be adsorbed onto the bead surface. The albumin and dextran sulfate, which are both biocompatible polymer materials, can degrade in the body, and thus can solve problems caused by the non-degradation of conventional beads using polyvinylalcohol in the body, for example, polyvinylalcohol is irregularly diffusd, causing an inflammation, or goes down the blood vessel and spreads into another organ, causing cerebrovascular disease.

As used herein, the term "biodegradable" refers to being capable of degrading when exposed to a physiological solution, and for example, refers to being capable of degrading by the body fluid or microorganisms in the living bodies of mammals including a human being.

According to an embodiment of the present invention, the albumin is a protein which is widely distributed in the body fluid, and includes animal albumins and vegetable albumins.

In one specific embodiment, the animal albumins include ovalbumin, serum albumin, lactalbumin, and miogen, and the vegetable albumins include leucosin (barely seeds), legumelin (peas), and lysine (castor seeds). The albumin includes albumin variants.

According to one embodiment of the present invention, the cross-linkage of the albumin is performed by thermal cross-linkage. As verified in the following examples, the microbeads, in which albumin was cross-linked, had higher anticancer drug adsorptivity than the beads prepared using glutaraldehyde as a cross-linking agent; had excellent body compatibility due to the non-use of a cross-linking agent which may be harmful to the body; and had economic advantages due to the omission of a cross-linking agent removing step (see tables 2 to 4). Cross-linkage of albumin can be performed by an aldehyde cross-linking agent. Aldehyde based cross-linking agent can be selected from the group consisting of glutaraldehyde, formaldehyde, dialdehyde starch, succinate aldehyde, acryl aldehyde, oxalaldehyde, 2-methylacrylaldehyde, and 2-oxopropanal.

According to one embodiment of the present invention, the anticancer drug adsorptivity of the microbeads of the present invention is 10-100 mg per 1 ml of microbeads. The anticancer drug adsorptivity of the microbeads of the present invention is 20-60 mg per 1 ml of microbeads for one specific embodiment, 20-55 mg per 1 ml of microbeads for another specific embodiment, and 20-50 mg per 1 ml of microbeads for still another specific embodiment.

According to one embodiment of the present invention, the anticancer drug adsorptivity of the microbeads of the present invention is improved by 20-45% of the amount of anticancer drug adsorbed on microbeads prepared in the same conditions except that a glutaraldehyde cross-linking agent is used instead of thermal cross-linkage. The anticancer drug adsorptivity of the microbeads of the present invention is improved by 21-43% for one specific embodiment, 22-42% for another specific embodiment, and 23-41% for still another specific example.

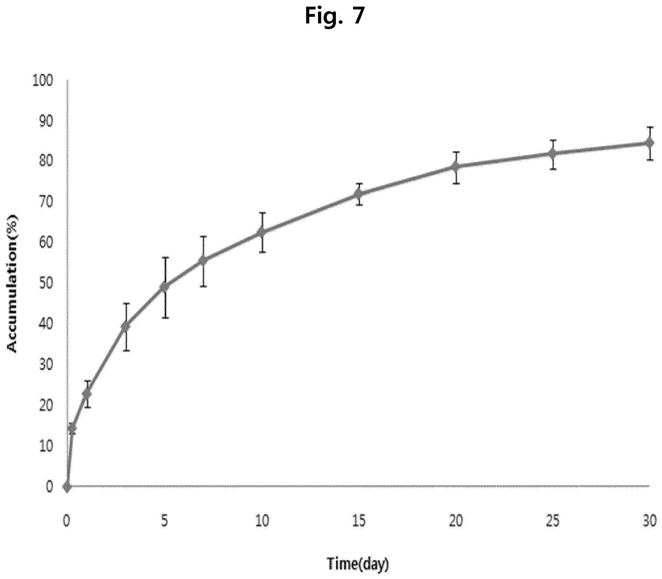

According to one embodiment of the present invention, the microbeads of the present invention exhibit a sustained release property. The following examples verified that doxorubicin was slowly released from the microbeads of the present invention over one month (see FIG. 7).

The microbeads of the present invention may be packaged in a vial together with a solution (wet microbead type), and selectively pulverized for the use (dry microbead type).

In accordance with another aspect of the present invention, there is provided a method for preparing biodegradable microbeads with improved anticancer drug adsorptivity, the method including:
(a) emulsifying a solution for preparing beads, in which albumin and dextran sulfate as an anionic polymer are dissolved, to form micro-sized bubbles; and
(b) thermally cross-linking the micro-sized bubbles in step (a) to form microbeads in which albumin is cross-linked and dextran sulfate is included in the albumin cross-linked product.

According to one embodiment of the present invention, the method of the present invention may further include, after step (b), (c) bringing the microbeads in step (b) into contact with an anticancer drug to allow the anticancer drug to be adsorbed onto surfaces of the microbeads by an electrostatic attraction of the dextran sulfate of the microbeads.

According to one embodiment of the present invention, the composition ratio of albumin and dextran sulfate in the solution for preparing beads in step (a) is 15-50:10% (W/V). In cases where the amount of albumin is significantly smaller than that of dextran sulfate in the solution for preparing beads, the beads are not strongly formed. In cases where the amount of dextran sulfate is significantly smaller than that of albumin, the anticancer drug adsorptivity deteriorates.

The composition ratio of albumin and dextran sulfate in the solution for preparing beads in step (a) is 15-45:10% (W/V) for one specific embodiment, 15-40:10% (W/V) for another specific embodiment, 15-35:10% (W/V), 5-30:10% (W/V) for still another specific embodiment, 20-45:10% (W/V) for still another specific embodiment, 20-40:10% (W/V) for still another specific embodiment, 20-35:10% (W/V) for still another specific embodiment, and 20-30:10% (W/V) for still another specific embodiment.

According to one embodiment of the present invention, the emulsification of the solution for preparing beads in step (a) is performed using an organic solvent containing natural oil or a viscosity-increasing agent. Examples of usable natural oil may be MCT oil, cottonseed oil, corn oil, almond oil, apricot oil, avocado oil, babassu oil, chamomile oil, canola oil, cocoa butter oil, coconut oil, cod-liver oil, coffee oil, fish oil, flax seed oil, jojoba oil, gourd oil, grape seed oil, hazelnut oil, lavender oil, lemon oil, mango seed oil, orange oil, olive oil, mink oil, palm tree oil, rosemary oil, sesame oil, shea butter oil, bean oil, sunflower oil, walnut oil, and the like.

Examples of the usable organic solvent may be acetone, ethanol, butyl acetate, and the like. The organic solvent may include a viscosity-increasing agent for providing appropriate viscosity. Examples of the viscosity-increasing agent may be cellulose based polymers, such as hydroxymethyl cellulose, hydroxypropyl methyl cellulose, and cellulose acetate butyrate. Preferably, the organic solvent containing the viscosity-increasing agent is butyl acetate containing cellulose acetate butyrate.

According to one embodiment of the present invention, the micro-sized bubbles in step (a) may be formed using a microfluidic system or an encapsulator. The microfluidic system is a method wherein beads are prepared using a micro-structured chip. After a smaller tube is positioned inside a larger tube, an aqueous phase and an oil phase are allowed to flow through the tubes in opposite directions, thereby forming beads by tension thereof. That is, when the solution for preparing beads as an inner fluid and the natural oil or organic solvent (collection solution) as an outer fluid are allowed to flow, the beads are formed by tension. The beads are collected into the collection solution, and then the beads may be prepared through a thermal cross-linkage reaction.

The encapsulation is similar to electrospinning, and is characterized in that an electric field, which is formed between a nozzle and a collection solution, finely splits water drops generated by tension, thereby dispersing very small-sized droplets. The solution for preparing beads is transferred into a syringe corresponding to the volume thereof, and the syringe is mounted on a syringe pump, and then connected with an encapsulator. In addition, the collection solution is transferred into a dish corresponding to the volume thereof, and then positioned on a stirrer. The environment of the encapsulator is appropriately set, and then the solution for preparing beads is sprayed to the collection solution to form bubbles. Preferably, the conditions of the encapsulator are preferably a flow rate of 1-5 ml/min, applied electric power of 1,000-3,000 V, ultrasonic wave of 2,000-6,000 Hz, and a revolution number of 100 rpm. The size of a release nozzle is selected according to the size of beads to be prepared.

According to another embodiment of the present invention, the micro-sized bubbles in step (a) may be prepared by an emulsifying method wherein a solution for preparing beads is mixed with a collection solution, and then the mixture is stirred at a proper revolution number. Here, the size of the beads depends on the revolution number and the stirring time. When appropriate-sized bubbles are formed, the collection solution is heated, thereby forming microbeads through thermal cross-linkage of albumin.

According to an embodiment of the present invention, the stirring continues to maintain a cross-linkage reaction of albumin until the cross-linkage reaction of albumin is completed, and upon completion of the reaction, the beads are washed several times using a large amount of acetone or ethanol for the washing of the collection solution.

According to one embodiment of the present invention, in step (b) of the present invention, the micro-sized bubbles obtained in step (a) are heated, so that albumin is thermally cross-linked to form a shape of a microbead and dextran sulfate is included in the thermally cross-linked product of albumin.

According to one embodiment of the present invention, the cross-linking temperature is 60-160°C and the cross-linking time is 1 to 4 hours. In one specific embodiment, the cross-linking temperature is 80-160°C and the cross-linking time is 1 to 4 hours.

According to one embodiment of the present invention, in cases where albumin is thermally cross-linked, the amount of anticancer drug adsorbed on the microbeads is increased by 20-45% as compared with microbeads prepared in the same conditions except that a glutaraldehyde cross-linking agent is used instead of thermal cross-linkage. The amount of anticancer drug adsorbed on the microbeads is increased by 21-43% for one specific embodiment, 22-42% for another specific embodiment, and 23-41% for still another specific example.

In accordance with still another aspect of the present invention, there is provided a method for treating cancer, the method including administering to a patient, biodegradable microbeads with improved anticancer drug adsorptivity, the microbeads including albumin which is cross-linked to form a shape of a bead; dextran sulfate, as an anionic polymer, included in the albumin cross-linked product; and an anticancer drug adsorbed on a bead surface by an electrostatic attraction with the anionic polymer.

According to the present invention, the microbeads of the present invention are administered into a cancer patient, thereby treating cancer through chemoembolization.

According to one embodiment of the present invention, the patient is a liver cancer patient, and the microbeads are administered to the hepatic artery of the patient.

### Advantageous Effects

The features and advantages of this invention will be summarized as follows:
(i) The present invention provides biodegradable microbeads with improved anticancer drug adsorptivity, and a method for preparing the same, and a method for treating cancer using the same.
(ii) The microbeads of the present invention are safe to the human body since the microbeads are prepared as a biocompatible and biodegradable polymer, and can effectively inhibit the growth of tumors by effectively blocking the blood vessel which supplies nutrition to the liver tumor and continuously releases an anticancer drug adsorbed onto the surfaces of the beads.
(iii) The present invention can prepare microbeads through thermal cross-linkage. The microbeads have higher anticancer drug adsorptivity compared with the microbeads prepared using a chemical cross-linking agent, have excellent body compatibility due to the non-use of a cross-linking agent, which may be harmful to the body, and have economic advantages due to the omission of a cross-linking agent removing step.
(iv) Therefore, the present invention can be favorably utilized for chemoembolization for liver cancer.

### Brief Description of the Drawings

FIG. 1 is a schematic view of a self-manufactured microfluidic system.
FIG. 2 shows images of microbeads prepared by the microfluidic system according to composition ratio 1.
FIG. 3 shows images of beads prepared by an encapsulator according to composition ratio 1.
FIG. 4 shows images of beads prepared by an emulsifying method according to composition ratio 1.

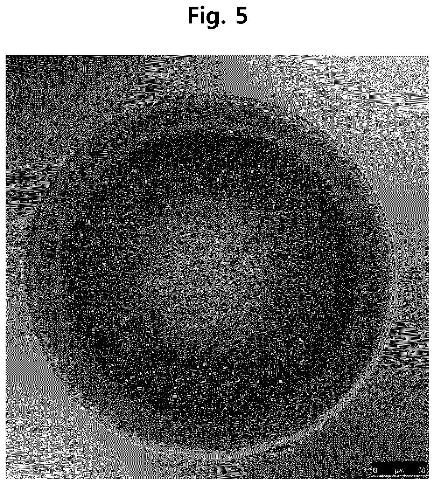

FIG. 5 is a cross-sectional view of a doxorubicin-adsorbed albumin/dextran sulfate bead.
FIG. 6 is a graph showing a short-term release behavior of doxorubicin-adsorbed albumin beads not containing dextran sulfate.
FIG. 7 is a graph showing a long-term release behavior of doxorubicin-adsorbed albumin/dextran sulfate beads.

### Mode for Carrying Out the Invention

The present invention will now be described in further detail by examples. It would be obvious to those skilled in the art that these examples are intended to be more concretely illustrative and the scope of the present invention as set forth in the appended claims is not limited to or by the examples.

### EXAMPLES

### Preparative Example: Preparation of Microbeads

### 1. Composition of Microbeads

The compositions of albumin and anionic polymer for preparing microbeads were shown in table 1 below.

**[Table 1]**

| W/V % | | Composition 1 | Composition 2 | Composition 3 | Composition 4 | Composition 5 |
|---|---|---|---|---|---|---|
| Albumin | Human serum albumin | 20 | 30 | - | - | 10 |
| | Bovine serum albumin | - | - | 20 | 30 | 10 |
| Anionic polymer | Dextran sulfate | 10 | 10 | 10 | 10 | 10 |

### 2. Preparation of Beads using Microfluidics

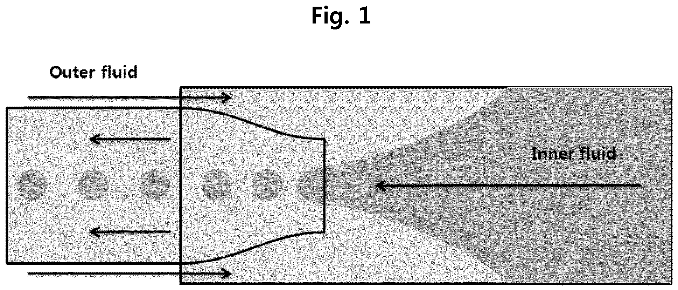

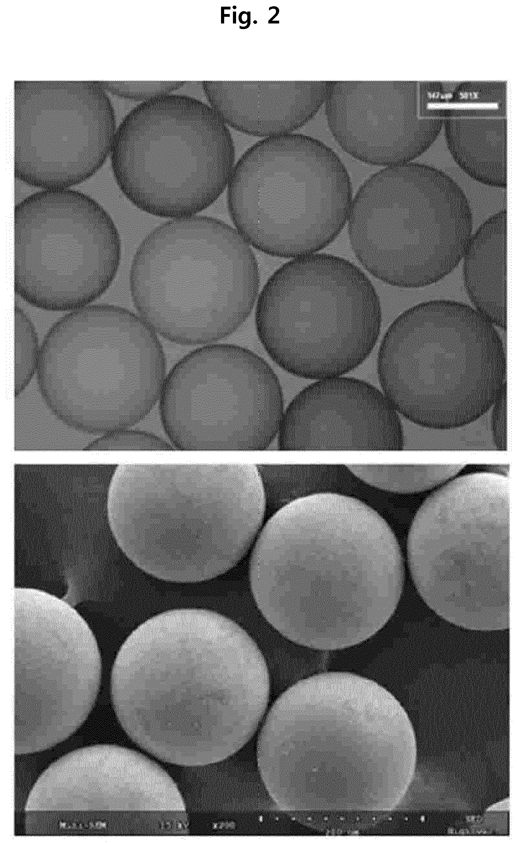

As shown in FIG. 1, solutions having compositions 1 to 5 above, as inner fluids, are allowed to flow at a flow rate of 2 ml/h through a self-produced microfluidic system, and MCT oil, which is a collection oil, as an outer fluid, is allowed to flow at a flow rate of 10 ml/h, thereby forming beads. Then, the formed beads are collected in a collection solution containing glutaraldehyde as a cross-linking agent, followed by a stirring reaction for 24 hours. FIG. 2 shows images of microbeads prepared by the microfluidic system according to composition ratio 1.

### 3. Preparation of Beads for Chemoembolization using Encapsulator

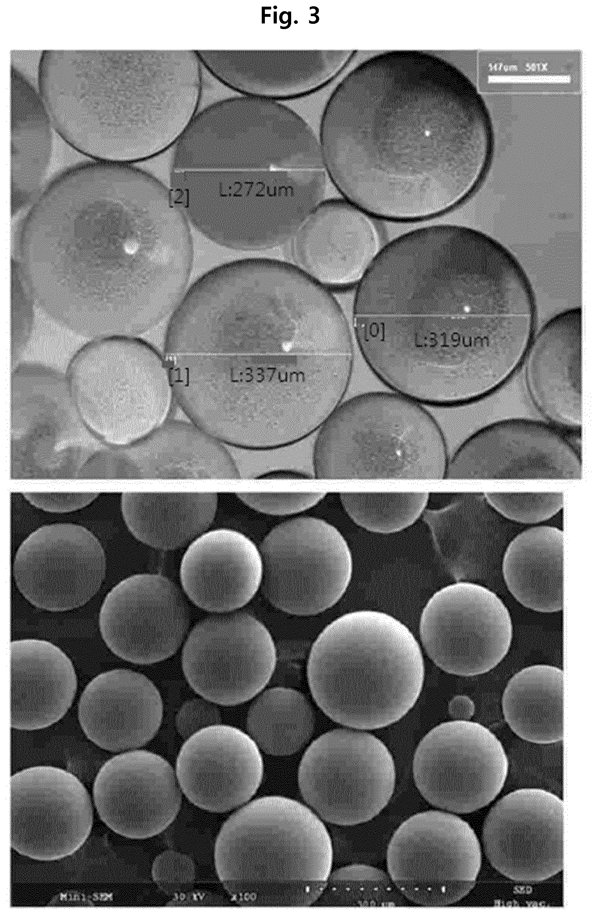

Beads with compositions 1 to 5 above were prepared using an encapsulator (B-390, BUCHI). The preparation conditions were: a flow rate of 1 to 5 ml/min, applied electric power of 1,000-3,000 V, ultrasonic wave of 2,000-6,000 Hz, and a revolution number of 100 rpm. The size of a release nozzle was selected according to the size of beads to be prepared. The prepared beads were collected in a collection solution, and then stirred for 24 hours. As the collection solution, butyl acetate containing 5-10% cellulose acetate butyrate or acetone containing propyl methylcellulose was used. FIG. 3 shows images of the microbeads prepared by the microfluidic system according to composition ratio 1.

### 4. Preparation of Beads for Chemoembolization using Encapsulator

Beads were formed under the same compositions and conditions as example 3 except that the cross-linking agent was not used and the collection solution was heated to 120°C to thermally cross-link albumin, which is a protein, thereby forming beads. The reaction time was 2 hours.

### 5. Preparation of Beads for Chemoembolization using Emulsification

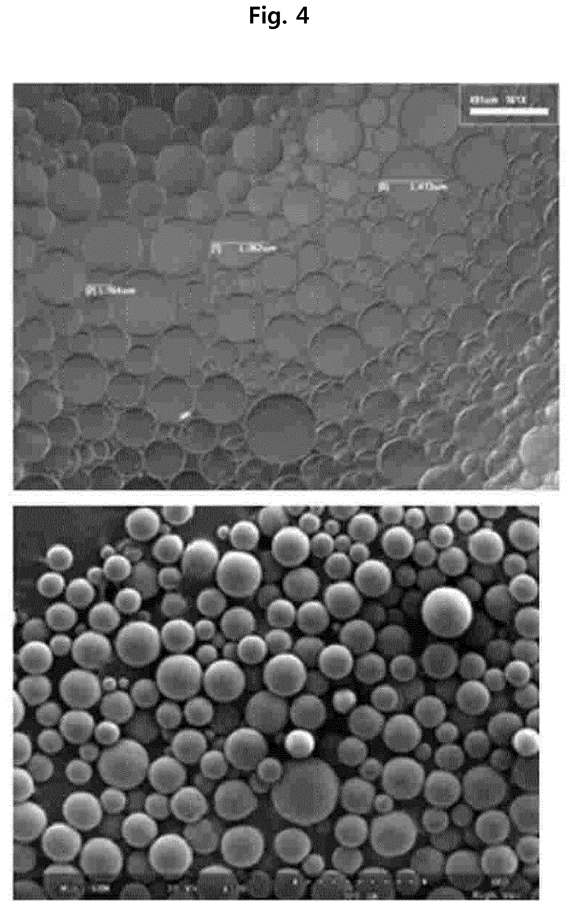

Solutions having compositions 1 to 5 above were mixed with a collection solution, followed by stirring. Simultaneously with the stirring, glutaraldehyde as a cross-linking agent was added. The reaction was conducted for 24 hours. As the collection solution, butyl acetate containing 5-10% cellulose acetate butyrate or acetone containing propyl methylcellulose was used. FIG. 4 shows images of the beads prepared by the emulsification method according to composition ratio 1.

### Test Example 1: Doxorubicin adsorption test

The doxorubicin adsorption test was conducted as follows. First, 50 mg of doxorubicin was dissolved in 2 ml of distilled water. Then, 1 ml of beads were taken, and put in a doxorubicin solution, followed by mixing well. After the mixture was left at room temperature for 20 minutes, the supernatant was taken, and then the absorbance at 483 nm was measured by an ultraviolet spectrometer. The amount of doxorubicin leaking out from 50 mg/2 ml of the doxorubicin solution may be determined by calculating the concentration through the comparison with the previously prepared calibration curve, and such a value was the amount of doxorubicin adsorbed on the beads. The test results are shown in table 2.

**[Table 2]**

| | | |
|---|---|---|
| Albumin : Dextran sulfate | 20:10 | 30:10 |
| Thermal cross-linkage | 45±1 mg/ml | 33±1 mg/ml |
| Glutaraldehyde cross-linkage | 34±1 mg/ml | 25±1 mg/ml |

As shown in table 2, the doxorubicin adsorption amounts of beads having compositions 1 and 3 were 33-35 mg/ml for cross-linkage using glutaraldehyde, ethyldimethylaminopropylcarbodimide (EDC), and N-hydroxysuccimide (NHS), but 44-46 mg/ml for protein denaturation through the application of heat. In addition, the doxorubicin adsorption amounts of beads having compositions 2 and 4 were 24-26 mg/ml for cross-linkage with glutaraldehyde, but 32-34 mg/ml for protein denaturation through the application of heat.

As a result of testing the daunorubicin and epirubicin adsorption amounts by the same method, the daunorubicin and epirubicin adsorption amounts were verified to be equivalent to the doxorubicin adsorption amounts (table 3).

**[Table 3]**

| Classification | Doxorubicin | | Daunorubicin | | Epirubicin | |
|---|---|---|---|---|---|---|
| Albumin: Dextran sulfate | 20:10 | 30:10 | 20:10 | 30:10 | 20:10 | 30:10 |
| Thermal cross-linkage (mg/ml) | 45±1 | 33±1 | 44±1 | 31±1 | 43±1 | 32±1 |
| Glutaraldehyde cross-linkage (mg/ml) | 34±1 | 25±1 | 32±1 | 24±1 | 33±1 | 23±1 |

### Test Example 2: Verification of residual glutaraldehyde

The residual glutaraldehyde after cross-linkage was neutralized with 5% sodium hydrogen sulfite. Here, in order to verify the residual amount of glutaraldehyde according to the number of times of treatment, the treatment with sodium hydrogen sulfite was conducted for different numbers of times, 0, 1, and 5 times, and for 30 minutes for each time. After that, the residual glutaraldehyde was confirmed at 483 nm using HPLC. The doxorubicin adsorption amount for each group was also measured. The test results are shown in table 4.

**[Table 4]**

| | No neutralization | One time of neutralization | Five times of neutralization |
|---|---|---|---|
| Residual glutaraldehyde (µg/ml) | 113.6 | 22.2 | 14.6 |
| Adsorbed doxorubicin (mg/ml) | 44.6±0.3 | 38.7±0.2 | 38.2±0.4 |

As can be verified in table 4, as the neutralization was repeated, the residual amount of glutaraldehyde decreased, but the doxorubicin adsorption amount also decreased. The inflow of the residual glutaraldehyde into the body may cause a risk of tissue fibrosis. However, as can be verified in test example 1, when beads are prepared by thermally cross-linking albumin through heating, there is no danger of the residual glutaraldehyde and the adsorptivity was improved (42-46 mg/ml of doxorubicin adsorption). The above adsorptivity is far higher than the doxorubicin adsorptivity (37 mg/ml) of DC beads of Biocompatible UK, which are currently commercialized and purchased. In addition, when the beads are mass-produced on an industrial scale, the glutaraldehyde neutralization process is not needed, thereby simplifying the process.

### Test Example 3: Doxorubicin release test

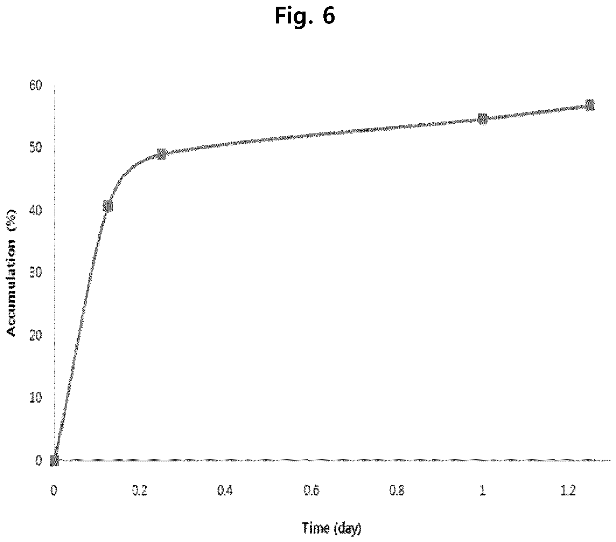

Doxorubicin release was verified for two groups. First, beads were fundamentally divided into an albumin/dextran sulfate bead group for verifying the release behavior of beads and a sulfate-non-containing albumin bead group for verifying the influence of dextran sulfate as an anionic polymer. The test method was as follows. Beads corresponding to 3.5 mg of doxorubicin were put in a 50 ml conical tube, which was filled with 50 ml of a release solution (PBS, pH 7.4), followed by incubation at 37°C. The release solution was all collected at the time of collection, and then exchanged with a new release solution. The release curve was calculated as an accumulative value. The released drug was analyzed by HPLC. The release results were shown in FIGS. 6 and 7.

As shown in FIG. 6, doxorubicin was also adsorbed onto albumin beads not containing dextran sulfate due to polarity of protein itself, but the release behavior thereof was very fast. Whereas, as shown in FIG. 7, the electrostatic attraction of the albumin beads containing dextran sulfate was stronger, and thus doxorubicin was slowly released over one month.

## Claims

1. Biodegradable microbeads, the microbeads comprising:
(i) albumin which is cross-linked by thermal cross-linkage to form a shape of a bead; and
(ii) dextran sulfate, as an anionic polymer, included in the albumin cross-linked product.

2. The microbeads of claim 1, further comprising an anticancer drug adsorbed on a bead surface by an electrostatic attraction with the anionic polymer.

3. The microbeads of claim 2, wherein the anticancer drug is an anthracycline based anticancer drug.

4. The microbeads of claim 3, wherein the anthracycline based anticancer drug is selected from the group consisting of daunorubicin, doxorubicin, epirubicin, idarubicin, gemcitabine, mitoxantrone, pirarubicin, and valrubicin.

5. The microbeads of claim 2, wherein the anticancer drug is irinotecan.

6. The microbeads of claim 1, which have an anticancer drug adsorptivity of 10-100 mg per 1 ml of microbeads.

7. Biodegradable microbeads, the microbeads comprising:
(i) albumin which is cross-linked by thermal cross-linkage to form a shape of a bead; and
(ii) dextran sulfate, as an anionic polymer, included in the albumin cross-linked product;
for use in chemoembolization.

8. The microbeads for the use of claim 7, wherein the chemoembolization is chemoembolization for liver cancer.

9. A method for preparing biodegradable microbeads, the method comprising:
(a) emulsifying a solution for preparing beads, in which albumin and dextran sulfate as an anionic polymer are dissolved, to form micro-sized bubbles; and
(b) thermally cross-linking the micro-sized bubbles in step (a) to form microbeads in which albumin is cross-linked and dextran sulfate is included in the albumin cross-linked product.

10. The method of claim 9, further comprising, after step (b), (c) bringing the microbeads in step (b) into contact with an anticancer drug to allow the anticancer drug to be adsorbed onto surfaces of the microbeads by an electrostatic attraction of the dextran sulfate of the microbeads.

## Patentansprüche

1. Biologisch abbaubare Mikrokügelchen, wobei die Mikrokügelchen umfassen:
(i) Albumin, das durch thermale Quervernetzung so quervernetzt ist, dass es eine Form eines Kügelchens bildet; und
(ii) Dextransulfat als anionisches Polymer, das in dem quervernetzten Albumin-Produkt enthalten ist.

2. Mikrokügelchen nach Anspruch 1, ferner umfassend ein Antikrebs-Arzneimittel, das auf einer Kügelchenoberfläche durch eine elektrostatische Anziehung mit dem anionischen Polymer adsorbiert ist.

3. Mikrokügelchen nach Anspruch 2, wobei das Antikrebs-Arzneimittel ein Anthracyclin-basiertes Antikrebs-Arzneimittel ist.

4. Mikrokügelchen nach Anspruch 3, wobei das Anthracyclin-basierte Antikrebs-Arzneimittel ausgewählt ist aus der Gruppe bestehend aus Daunorubicin, Doxorubicin, Epirubicin, Idarubicin, Gemcitabin, Mitoxantron, Pirarubicin und Valrubicin.

5. Mikrokügelchen nach Anspruch 2, wobei das Antikrebs-Arzneimittel Irinotecan ist.

6. Mikrokügelchen nach Anspruch 1, welche eine Antikrebs-Arzneimittel-Adsorptionsfähigkeit von 10-100 mg pro 1 ml Mikrokügelchen aufweisen.

7. Biologisch abbaubare Mikrokügelchen, wobei die Mikrokügelchen umfassen:
(i) Albumin, das durch thermale Quervernetzung so quervernetzt ist, dass es eine Form eines Kügelchens bildet; und
(ii) Dextransulfat als anionisches Polymer, das in dem quervernetzten Albumin-Produkt enthalten ist,
zur Verwendung bei Chemoembolisation.

8. Mikrokügelchen zur Verwendung nach Anspruch 7, wobei die Chemoembolisation Chemoembolisation für Leberkrebs ist.

9. Verfahren zur Herstellung von biologisch abbaubaren Mikrokügelchen, wobei das Verfahren umfasst:
(a) Emulgieren einer Lösung zur Herstellung von Kügelchen, in der Albumin und Dextransulfat als ein anionisches Polymer gelöst sind, zur Bildung von mikrogroßen Blasen; und
(b) thermales Quervernetzen der mikrogroßen Blasen in Schritt (a) zur Bildung von Mikrokügelchen, in denen Albumin quervernetzt ist und Dextransulfat in dem quervernetzten Albumin-Produkt enthalten ist.

10. Verfahren nach Anspruch 9, ferner umfassend nach Schritt (b), (c) In-Kontakt-Bringen der Mikrokügelchen in Schritt (b) mit einem Antikrebs-Arzneimittel, um zu ermöglichen, dass das Antikrebs-Arzneimittel auf Oberflächen der Mikrokügelchen durch eine elektrostatische Anziehung des Dextransulfats der Mikrokügelchen adsorbiert wird.

## Revendications

1. Microbilles biodégradables, les microbilles comprenant :
(i) une albumine qui est réticulée par réticulation thermique pour prendre la forme d'une bille ; et
(ii) du sulfate de dextran, en tant que polymère anionique, inclus dans le produit d'albumine réticulé.

2. Microbilles selon la revendication 1, comprenant en outre une substance médicamenteuse anticancéreuse adsorbée sur une surface des billes par une attraction électrostatique avec le polymère anionique.

3. Microbilles selon la revendication 2, dans lesquelles la substance médicamenteuse anticancéreuse est une substance médicamenteuse anticancéreuse à base d'anthracycline.

4. Microbilles selon la revendication 3, dans lesquelles la substance médicamenteuse anticancéreuse à base d'anthracycline est choisie dans le groupe constitué par la daunorubicine, la doxorubicine, l'épirubicine, l'idarubicine, la gemcitabine, la mitoxantrone, la pirarubicine et la valrubicine.

5. Microbilles selon la revendication 2, dans lesquelles la substance médicamenteuse anticancéreuse est l'irinotécan.

6. Microbilles selon la revendication 1, qui ont une adsorptivité de substance médicamenteuse anticancéreuse de 10 à 100 mg pour 1 ml de microbilles.

7. Microbilles biodégradables, les microbilles comprenant :
(i) une albumine qui est réticulée par réticulation thermique pour prendre la forme d'une bille ; et
(ii) du sulfate de dextran, en tant que polymère anionique, inclus dans le produit d'albumine réticulé ;
pour l'utilisation en chimio-embolisation.

8. Microbilles pour l'utilisation selon la revendication 7, dans lesquelles la chimio-embolisation est une chimio-embolisation pour un cancer du foie.

9. Procédé de préparation de microbilles biodégradables, le procédé comprenant :
(a) l'émulsification d'une solution pour préparer des billes, dans laquelle de l'albumine et du sulfate de dextran en tant que polymère anionique sont dissous, pour former des micro-bulles ; et
(b) la réticulation thermique des micro-bulles de l'étape (a) pour former des microbilles dans lesquelles l'albumine est réticulée et le sulfate de dextran est inclus dans le produit d'albumine réticulé.

10. Procédé selon la revendication 9, comprenant en outre, après l'étape (b), (c) la mise en contact des microbilles de l'étape (b) avec une substance médicamenteuse anticancéreuse pour permettre à la substance médicamenteuse anticancéreuse d'être adsorbée sur les surfaces des microbilles par une attraction électrostatique du sulfate de dextran des microbilles.
